**Europäisches Patentamt**

**European Patent Office**

**Office européen des brevets**

(19)

(11) Veröffentlichungsnummer: **0 191 297 B1**

(12) **EUROPÄISCHE PATENTSCHRIFT**

(45) Veröffentlichungstag der Patentschrift: **30.05.90**

(21) Anmeldenummer: **86100260.8**

(22) Anmeldetag: **10.01.86**

(51) Int. Cl.⁵: **A 61 F 7/00**, A 61 B 17/36, F 16 L 11/12

(54) **Mit einer Isolation versehener Kunststoffschlauch.**

(30) Priorität: **14.02.85 DE 3505045**

(43) Veröffentlichungstag der Anmeldung:
**20.08.86 Patentblatt 86/34**

(45) Bekanntmachung des Hinweises auf die
Patenterteilung:
**30.05.90 Patentblatt 90/22**

(84) Benannte Vertragsstaaten:
**AT BE CH FR GB IT LI NL SE**

(56) Entgegenhaltungen:
**DE-A-2 422 103**
**DE-A-2 435 443**
**DE-A-3 305 434**
**DE-B-1 922 487**
**US-A-3 908 655**

(73) Patentinhaber: **MESSER GRIESHEIM GMBH**
**Hanauer Landstrasse 330**
**D-6000 Frankfurt/Main (DE)**

(72) Erfinder: **Donnerhack, Andreas, Dr.**
**Bismarckstrasse 4**
**D-4150 Krefeld (DE)**
Erfinder: **Jankowski, Detlef**
**Kortumstrasse 127**
**D-4100 Duisburg 1 (DE)**
Erfinder: **Pfeil-Schneider, Kurt**
**Johannesfeld 29**
**D-4054 Nettertal (DE)**
Erfinder: **Thoma, Klemens**
**Am Kleckers 22**
**D-4150 Krefeld-Hüls 29 (DE)**
Erfinder: **Volker, Wolfgang**
**Pastorsbusch 35**
**D-4154 Tönisvorst 1 (DE)**

**Beschreibung**

Die Erfindung betrifft einen mit einer Isolation versehenen Kunststoffschlauch nach dem Oberbegriff des Anspruches 1 wie er aus der DE—A— 2 435 443 bekannt ist.

Für die Rheumabehandlung hat in letzter Zeit die Kryotherapie an Bedeutung gewonnen. Hierbei wird ein vorzugsweise trockener Gasstrom von Temperaturen bis zu −180°C auf den zu behandelnden Gelenken oder Körperpartien appliziert. Dieser Gasstrom besteht je nach Funktionsprinzip des jeweiligen Gerätes aus kalter Luft, kaltem Stickstoffgas oder einer Mischung aus beiden. Um diesen Kaltgasstrom an den Patienten heranführen zu können und auf die zu behandelnden Körperpartien richten zu können, ist ein Schlauch erforderlich, der eine Reihe von speziellen Anforderungen erfüllen muß.

Zunächst einmal muß der Schlauch so gut thermisch isoliert sein, daß beim Durchströmen des bis zu −180°C kalten Gases keine erhebliche Abkühlung der äußeren Oberfläche des Schlauches erfolgt. Die Isolation muß so effektiv sein, daß sich im Dauerbetrieb, auch bei hoher Raumfeuchte, praktisch kein Kondenswasser absetzt. Durch eine gute Isolation wird gleichzeitig auch eine wirksame Ausnutzung des Kältemediums erreicht. Trotz dieser guten Isolation soll der Schlauch auch bei Betriebstemperatur noch über eine ausreichende Flexibilität verfügen, so daß der Therapeut ohne den Patienten zu bewegen, die betreffenden Körperregionen gleichmäßig und bequem mit dem Kaltgasstrahl erreichen kann. Trotz dieser hohen Flexibilität muß der Schlauch jedoch auch über eine ausreichende mechanische Stabilität verfügen. Auch das Gewicht des Schlauches darf nicht zu groß werden, da bei zu hohem Gewicht ebenfalls die bequeme Handhabung beeinträchtigt sein würde. Ferner ist es wünschenswert, daß die Kältekapazität des Schlauches gering ist, um die Einkühlzeit und die Kältemittelverluste klein zu halten. Ferner ist es wünschenswert, daß der Schlauch preiswert ist, also aus Standardmaterialien einfach montiert werden kann. Diese Forderungen gelten selbstverständlich auch für die Schlauchendstücke.

Die derzeit gebräuchlichen Schläuche erfüllen nicht die Gesamtheit dieser Forderungen. Eine in der Kryotechnik bewährte Isolation wird beispielsweise auf die Leitungen aufgeklebt. Bei tiefen Temperaturen wird sie aber hart, so daß sie für flexible Schäuche nicht zu gebrauchen ist. Werden dagegen die Schläuche durch Schüttungen von losen Isoliermaterial isoliert, so haben sie den Nachteil, daß das Isoliermaterial sich im laufe der Zeit in den tieferliegenden Bereichen ansammelt. Hier wird das Isolationsmaterial verdichtet, wodurch Änderungen der Isolationseigenschaften verursacht werden. Derartige Verdichtungen treten naturgemäß bei häufig bewegten Schläuchen, wie es in der Kryotherapie der Fall ist, besonders häufig auf. Superisolierte Schläuche sind zu teuer und haben einen zu großen Biegeradius. Einen zu großen Biegeradius haben auch Wellrohre und Glattrohre aus Kunststoff, abgesehen davon, daß sie noch zusätzlich isoliert werden müßten. Wellrohre aus Metall haben eine zu große Kältekapazität und sind schwer. Außerdem benötigen sie viel platz für die Isolation.

Der Erfindung liegt die Aufgabe zugrunde, einen insbesondere für die Kryotherapie geeigneten Behandlungsschlauch zu schaffen, der bei guter thermischer Isolation eine hohe Flexibilität auch bei Behandlungstemperatur, ein geringes Gewicht und eine geringe Kältekapazität besitzt, sowie zudem preiswert hinsichtlich Material und Herstellung ist.

Ausgehend von dem im Oberbegriff des Anspruches 1 berücksichtigten Stand der Technik ist diese Aufgabe erfindungsgemäß gelöst mit den im kennzeichnenden Teil des Anspruches 1 angegebenen Merkmalen.

Vorteilhafte Weiterbildungen der Erfindung sind in den Unteransprüchen angegeben.

Der erfindungsgemäße Schlauch besteht ausschließlich aus Kunststoffmaterialien und besitzt eine Isolation im Mehrschichtenaufbau. Für die einzelnen Schichten werden Schlauch- oder Rohrmaterialien verwendet. Dadurch wird die Montage einfach. Da ausschließlich Kunststoffmaterialien verwendet werden, wird die Kältekapazität und das Gewicht des Schlauches gering gehalten. Die einzelnen Schichten der Isolation sind dünn oder faserig, wodurch eine hohe Flexibilität ermöglicht wird. Lufteinschlüsse, insbesondere in den faserigen Schichten, erhöhen die thermische Isolation. Eine Aluminiumschicht reflektiert die von außen eindringende Wärmestrahlung.

Der Innenschlauch ist ein massiver Kunststoffschlauch, der somit eine ausreichende mechanische Stabilität besitzt. Ein solcher Innenschlauch ist jedoch an sich nicht flexibel genug, er wird deshalb als Spiralgliederschlauch ausgebildet. Da ein Spiralgliederschlauch nicht gasdicht ist, übernehmen die umhüllenden Schichten, insbesondere der Wellschlauch aus Glasseidengewebe, diese Aufgabe. Überraschend hat sich gezeigt, daß für den inneren Spiralgliederschlauch PVC als Material hervorragend geeignet ist, obwohl PVC bei tiefen Temperaturen nur als beschränkt verwendungsfähig gilt. Auch die Fertigung des erfindungsgemäßen Schlauches ist einfach und kann ohne zusätzliche Vorrichtungen oder Hilfsmittel durchgeführt werden.

Der erfindungsgemäße Kunststoffschlauch ist für den Einsatz in der Kryotherapie hervorragend geeignet. Er ist aber auf diesen Verwendungszweck nicht beschränkt, sondern kann überall dort eingesetzt werden, wo tiefkalte Gase durch flexible Leitungen strömen.

Die Zeichnungen veranschaulichen ein Ausführungsbeispiel der Erfindung im Schnitt, nämlich einen Behandlungsschlauch für die Kryotherapie.

Es zeigen:

Fig. 1 das maschinenseitige Endstück des Schlauches,

Fig. 2 das griffseitige Endstück des Schlauches mit einer aufsetzbaren Düse.

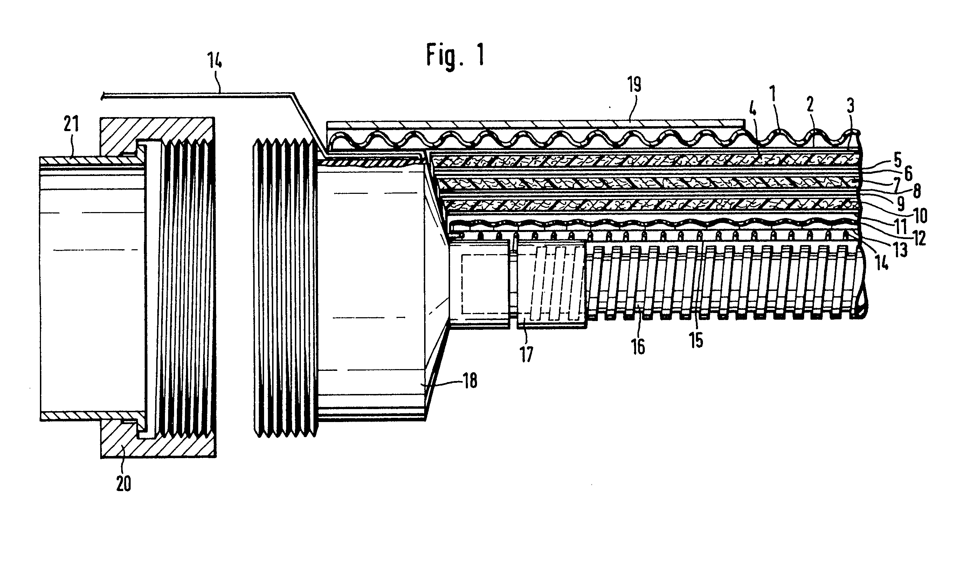

Bei dem in Fig. 1 dargestellten erfindungsgemäßen Kunststoffschlauch mit maschinenseitigem Endstück ist der eigentliche, dem Gastransport dienende Kunststoffschlauch der Spiralgliederschlauch 16. Er besteht aus Polyvinylchlorid (PVC). Da der Spiralgliederschlauch 16 nicht gasdicht ist, wird er von einem Wellschlauch 12 aus mit aluminiumbeschichteten Glasseidengewebe umgeben. Dieser Wellschlauch 12 ist ausreichend gasdicht. Die Aluminiumbeschichtung verhindert das Eindringen von Wärmestrahlung aus der Umgebung. Zwischen dem Spiralgliederschlauch 16 und dem Wellschlauch 12 sind zwei Folien 13, 15 aus Polyäthylenterephthalat (PET) angebracht. Zwischen den Folien 13, 15 befindet sich eine Kabelwicklung 14 zur Weiterleitung elektrischer Signale. Hierbei kann es sich beispielsweise um die Signale eines Temperaturfühlers handeln, der im Griffende des Behandlungsschlauches angeordnet ist. Sowohl die Folien 13, 15 als auch die Kabelwicklung 14 sind für die thermische und mechanische Funktion des Behandlungsschlauches nicht erforderlich.

Auf dem Wellschlauch 12 befindet sich eine weitere Folie 11 aus PET. Darüber sind zur weiteren Isolation drei Gruppen aus Kunststoffilz und Kunststoffolien angeordnet. Jede Gruppe besteht aus einem Polyesterfilz 4, 7, 10, einer inneren Folie 3, 6, 9 aus Polyäthylen (PE) und einer äußeren Folie 2, 5, 8 aus PET. Den äußeren Abschluß bildet ein Wellschlauch aus Polyesterelastomer.

Das maschinenseitige Endstück des erfindungsgemäßen Kunststoffschlauches stellt ein Schraubelement 18 aus PVC dar. Die Befestigung des Spiralgliederschlauches 16 an dem Schraubelement 18 erfolgt über ein Gewindeübergangsstück 17 aus PVC. Die Arretierung des äußeren Wellschlauches 1 auf dem Schraubelement 18 erfolgt durch Überziehen einer Metallhülse 19 aus vernickeltem Messing.

Durch eine Rinne im Schraubelement 18 wird die Kabelwicklung 14 nach außen geführt. Mittels der Überwurfmutter 20 mit das Schraubelement 18 and das maschinenseitige Rohrendstück 21 angeschraubt.

Die Gesamtdicke des erfindungsgemäßen Kunststoffschlauches ergibt sich aus den gewählten Dicken der jeweiligen Einzelschichten. Je mehr Gruppen von Kunststoffilzen und Kunststoffolien man zur Isolation aufbringt, um so besser wird die Isolationsqualität. Gleichzeitig verringert sich aber die Flexibilität des Schlauches. Eine Dreiergruppe aus Kunststoffilzen und Kunststoffolien hat sich als optimal erwiesen. Bei einem Schlauch mit beispielsweise 72mm Außendurchmesser sind dabei Krümmungsradien von ca. 50cm möglich.

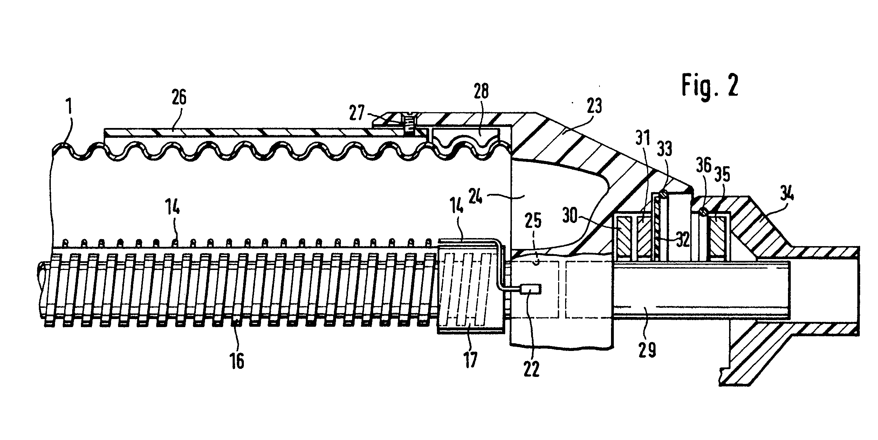

In Fig. 2 ist das griffseitige Endstück des Behandlungsschlauches dargestellt. Die Kabelwicklung 14 endet hierbei in einem Kontaktstück 22 eines Temperaturfühlers, der im Schlauchendstück 23 aus Polycarbonat angeordnet ist. Das Schlauchendstück 23 besitzt eine Aussparung 24 zur Verbesserung der Isolation. Außerdem wird durch die Aussparung 24 die Stabilität gegenüber thermischen Spannungen vergrößert. Das Schlauchendstück 23 besitzt ein Gewinde 25, in welches der Spiralgliederschlauch 16 eingeschraubt und mittels eines Gewindeübergangsstückes 17 arretiert ist. Mit einem entsprechenden Gewindeübergangstück 17 ist der Spiralgliederschlauch 16 im Schraubelement 18 maschinenseitig arretiert. Das Schalauchendstück 23 nimmt den äußeren Wellenschlauch 1 und ein darübergeschobenes Kunststoffrohr 26 auf. Das Kunststoffrohr 26 stellt die eigentliche Griffläche dar. Es wird über drei, über den Umfang verteilte Schrauben 27 mit dem Schlauchendstück 23 verbunden. Wahlweise kann diese Verbindung auch durch Verkleben erfolgen. Um die Einheit aus Kunststoffrohr 26 und Schlauchendstück 23 gegen den Wellschlauch 1 zu arretieren, werden vor der Montage zwei Halbschalen 28 über den Wellschlauch 1 gelegt, die über eine Breite von 2 bis 3 Gängen der Wellenform des Schlauches angepaßt sind.

In das Schlauchendstück 23 ist ferner ein Rohrstück 29 eingeschraubt. Um das Rohrstück 29 herum sind in Aussparungen im Schlauchendstück 23 ein Weicheisenring 30, ein ringförmiger Permanentmagnet 31 sowie eine Abdeckscheibe 32 aus Kunststoff eingesetzt. Sie werden durch eine Ringfeder 33 arretiert.

Die der Behandlung dienende Düse 34 kann auf das Rohrstück 29 aufgeschoben werden. Auch in der Düse 34 befindet sich ein ringförmiger Permanentmagnet 35, welcher durch eine Ringfeder 36 gehalten wird. Der Permanentmagnet 35 bildet den Gegenpol zu dem Permanentmagnet 31 und ermöglicht beim Zusammenführen der Düse 34 und des Schlauchendstückes 23 die Haftung beider Teile. Das Rohrstück 29 wird so lang gemacht, daß der Kaltgasstrom moglichst weit in die Düse 34 hineingeführt wird und Turbulenzen an nicht gasdichten Stellen vermieden werden.

Anstelle des ringförmigen Permanentmagneten 31 im Schlauchnsendstück 23 können auch Stiftmagneten eingebaut werden, wobei der ringförmige Permanentmagnet 35 in der Düse 34 beibehalten werden sollte. Bei ausreichender Stärke der Magnetkraft reicht als Gegenstück Weicheisenmaterial auch allein aus.

Die Düse 34 kann an ihrer vorderen Mündung mit Reduzierund Winkelstücken ergänzt werden. Durch die Art der Arretierung kann die Düse 34 leicht gedreht werden. Als Material für die Düse 34 dient kältebeständiger Kunststoff, auch hier hat sich überraschender Weise PVC als geeignet erwiesen.

**Patentansprüche**

1. Mit einer Isolation versehener Kunststoffschlauch für die Förderung tiefkalter Gase, dadurch gekennzeichnet, daß der Schlauch als Spiralgliederschlauch (16) ausgebildet ist, der von einem gasdichten Wellschlauch (12) aus mit Aluminium beschichtetem Glasseidengewebe umgeben ist, während die Isolation aus mehreren aufeinanderfolgenden Schichten aus Kunststoffolien und Kunststoffilzen gebildet wird, die von einem äußeren Wellschlauch (1) aus Kunststoff umgeben sind.

2. Kunststoffschlauch nach Anspruch 1, dadurch gekennzeichnet, daß die Isolation aus mehreren aufeinanderfolgenden Gruppen von Kunststoffolien und Kunststoffilzen besteht, wobei jede Gruppe aus einem Polyesterfilz (4, 7, 10), einer inneren Folie (3, 6, 9) aus Polyäthylen (PE) sowie einer äußeren Folie (2, 5, 8) aus Polyäthylenterephthalat (PET) besteht.

3. Kunststoffschlauch nach Anspruch 2, gekennzeichnet durch drei Gruppen von Kunststoffolien und Kunststoffilzen.

4. Kunststoffschlauch nach einem der Ansprüche 1 bis 3, dadurch gekennzeichnet, daß der Spiralgliederschlauch (16) aus Polyvinylchlorid (PVC) besteht.

5. Kunststoffschlauch nach einem der Ansprüche 1 bis 4, dadurch gekennzeichnet, dab der äubere Wellschlauch (1) aus Polyesterelastomer besteht.

6. Kunststoffschlauch nach einem der Ansprüche 1 bis 5, dadurch gekennzeichnet, daß zwischen Spiralgliederschlauch (16) und gasdichtem Wellschlauch (12) zwei PET-Folien (13, 15) mit einer dazwischen befindlichen Kabelwicklung (14) für die Weiterleitung elektrischer Signale angeordnet sind.

**Revendications**

1. Tuyau en matériau synthétique, muni d'une isolation pour le transport de gaz à très basse température, caractérisé en ce que, le tuyau est réalisé comme un tuyau à éléments hélicoïdaux (16), entoure d'un tuyau ondulé (12) etanche aux gaz en tissu de soie de verre recouvert d'aluminium, alors que l'isolation est formée de plusieurs couches successives de feuilles de matière synthétique et de feutres en matière synthétique, qui sont entourées par un tuyau extérieur ondulé (1) en matière synthétique.

2. Tuyau en matière synthétique selon la revendication 1, caractérisé en ce que, l'isolation se compose de plusieurs groupes successifs de feuilles de matière synthétique et de feutres de matière synthétique, chaque groupe comprenant outre un feutre de polyester (4, 7, 10), une feuille intérieure (3, 6, 9) en polyéthylène (PE) ainsi qu'une feuille extérieure (2, 5, 8) en polyéthylène-téréphtalate (PET).

3. Tuyau en matière synthétique selon la revendication 2, caractérisé par trois groupes de feuilles de matière synthétique et de feutres synthétiques.

4. Tuyau en matière synthétique selon une des revendications 1 à 3, caractérisé en ce que le tuyau en éléments hélicoïdaux (16) est constitué de chlorure de polyvinyle (PVC).

5. Tuyau en matière synthétique selon une des revendications 1 à 4, caractérisé en ce que le tuyau ondulé extérieur (1) est constitué d'élastomère de polyester.

6. Tuyau en matière synthétique selon une des revendications 1 à 5 caractérisé en ce qu'entre tuyau éléments hélicoïdaux (16) et tuyau ondulé (12) étanche aux gaz sont disposées deux feuilles PET (13, 15) avec dans leur intervalle un enroulement de câble (14) pour la transmission de signaux électriques.

**Claims**

1. Insulated plastic tube for conveying deep-cooled gases, characterized in that the tube is in the form of a spiral-articulated tube (16) which is surrounded by a gas-tight corrugated tube (12) composed of aluminium-coated glass fibre fabric, and the insulation is formed from a plurality of successive layers composed of plastic films and synthetic felts which are surrounded by an external corrugated tube (1) of plastic.

2. Plastic tube according to Claim 1, characterized in that the insulation is composed of a plurality of successive groups of plastic films and synthetic felts, each group being composed of a polyester felt (4, 7, 10), an internal film (3, 6, 9) of polyethylene (PE) and an external film (2, 5, 8) of polyethylene terephthalate (PET).

3. Plastic tube according to Claim 2, characterized by three groups of plastic films and synthetic felts.

4. Plastic tube according to one of Claims 1 to 3, characterized in that the spiral-articulated tube (16) is composed of polyvinyl chloride (PVC).

5. Plastic tube according to one of Claims 1 to 4, characterized in that the external corrugated tube (1) is composed of polyester elastomer.

6. Plastic tube according to one of Claims 1 to 5, characterized in that between the spiral-articulated tube (16) and the gas-tight corrugated tube (12), there are two PET films (13, 15) between which is a cable winding (14) for the transmission of electrical signals.

Fig. 1

Fig. 2